# EUROPEAN PATENT SPECIFICATION

(11) **EP 2 309 934 B1**
(45) Date of publication and mention of the grant of the patent: **07.11.2018**
(21) Application number: 08766822.4
(22) Date of filing: 19.06.2008
(51) Int. Cl.: A61B 17/22, A61B 17/00, A61B 17/3207

(54) **A THROMBECTOMY CATHETER AND A DEVICE COMPRISING THE SAME**
THROMBEKTOMIE-KATHETER UND DIESEN UMFASSENDE VORRICHTUNG
CATHÉTER DE THROMBECTOMIE ET DISPOSITIF COMPRENANT CELUI-CI

(43) Date of publication of application: 20.04.2011
(73) Proprietor: AngioDynamics, Inc., Latham, NY 12110 (US)
(72) Inventor: WITTENS, Cornelis Hendrikus Anna, 3065 DA Rotterdam (NL)
(74) Representative: Lloyd, Robin
(86) International application number: PCT/NL2008/050399
(87) International publication number: WO 2009/154441

(56) References cited:
- WO-A-2005/079678
- US-A1- 2003 055 445
- US-A1- 2004 082 962
- US-B1- 6 719 717

## Description

### FIELD OF THE INVENTION

The invention relates to a thrombectomy catheter comprising a distal end for at least partially removing thrombus from a blood vessel, said distal end being conceived to undergo displacement along the blood vessel. The invention further relates to a thrombectomy device.

### BACKGROUND OF THE INVENTION

Venous thrombosis, in particular, deep venous thrombosis is a disease with significant incidence in the Western world, affecting 1 - 2 per 1000 individual annually. When untreated, severe complications may occur, ranging from redness, swelling and pain, due to venous obstruction, to fatal pulmonary embolism.

Concurrently, preferable treatment of the venous thrombosis and/or deep venous thrombosis relates to minimally invasive endovascular interventions. For example, a thrombectomy catheter may be introduced in the affected vein to cause mechanical destruction of the thrombotic tissue and removal thereof from the vein.

US 2004/0082962 A1 discloses a catheter having a positioning cage and a macerator to achieve thrombectomy and other treatments.

An embodiment of a thrombectomy catheter is known from US 2003/0055445. The known thrombectomy catheter is arranged to at least partially remove obstructive material from vasculature and comprises an elongated body having a lumen propagating from a proximal end of the catheter to a sub-distal end of the catheter for receiving pieces of obstructive material destroyed by a morcellator arranged at a distal end of the catheter. The morcellator of the known thrombectomy catheter comprises a rotatable body arranged inside a static cage. The morcellator is provided as a suitable bundle of cooperating wires, which may together with the static cage be alternated from a substantially collapsed state to a substantially expanded state. The substantially collapsed state is practical for introducing the distal portion of the known thrombectomy catheter intravascularly. For purposes of collecting and destroying thrombotic tissue, the morcellator is expanded in use.

It is a disadvantage of the known thrombectomy catheter that substantially low efficiency of thrombosis removal is achievable. First, due to the fact that the rotating morcellator is arranged inside the outer static cage a substantially small volume is left between the morcellator and the surface of the cage for receiving thrombus. This may have an effect that the known thrombectomy catheter has to be substantially slowly displaced inside a blood vessel thereby increasing time necessary to complete the intervention. Secondly, due to the fact that the outer static cage is implemented as a wire-frame having openings throughout substantially the whole surface of the cage, it is possible that thrombus may exit the cage through such openings before it comes into contact with the morcellator. This may lead to a mere perforation of thrombus instead of removal thereof and, therefore, may further decrease efficacy of the known thrombectomy catheter necessitating the intervention to be repeated to pick up leftovers of thrombus.

### SUMMARY OF THE INVENTION

The invention is defined in the appended claims. Aspects, embodiments and examples disclosed of the present disclosure which do not fall under the scope of the appended claims do not form part of the invention and are merely provided for illustrative purposes.

It is an object of the invention to provide a thrombectomy catheter having an increased efficiency for removal of thrombus.

To this end a thrombectomy catheter according to the invention comprises a lumen being arranged for collecting thrombus and having a cross-section which is alterable from a collapsed state to an expanded state, wherein during displacement of the distal end the cross-section of the lumen is self-adjustable for substantially matching local cross-sectional dimensions of the blood vessel.

The technical measure of the invention is based on the insight that by providing a lumen with a controllable cross-section substantially at the distal end of the catheter, efficacy of removal of thrombus from the blood vessel is increased. In particular, the lumen may be arranged to be in a collapsed state for enabling due introduction of the catheter into the blood vessel. When it is established that the distal end of the thrombectomy catheter is positioned substantially nearby a thrombotic tissue, the lumen may be expanded to increase its cross-section and to cause an outer surface of the lumen to come into contact with the blood vessel. Under displacement of the catheter with the thus expanded lumen along the blood vessel the lumen may act as a scoop thereby removing the thrombotic tissue from a surface of the blood vessel. After completion of this procedure the lumen of the thrombectomy catheter may be again collapsed for simplifying its removal from the vasculature.

Preferably, the thrombectomy catheter according to the invention is formed by a tubular structure running from a proximal end of the catheter to the distal end of the catheter, the lumen forming integral part of said tubular structure. Thus, the expandable lumen acting as a scoop is integrated with the overall lumen of the catheter. Therefore, no extra manufacturing steps are required for providing such lumen. Preferably, the lumen is manufactured from a smooth material, like latex, or the like to ease its displacement along inner periphery of the blood vessel.

It has been found that a substantial decrease of time required for intervention is achieved with the thrombectomy catheter according to the invention. For example, it may be possible to successfully accomplish thrombectomy of at least axillary, brachial, iliac, femoral, tibial, popliteal, great saphenous, small saphenous, jugual and basilic veins within about half an hour.

In addition, the thrombectomy catheter according to the invention is particularly suited for treating veins located in the lower extremities, as during collecting of thrombus the catheter is conceived to be uni-directionally displaced in a direction not compromising the vascular valves, thereby causing no disruption thereof. When the thrombectomy treatment is accomplished, the catheter's lumen may be collapsed and the thrombectomy catheter may be removed from the vasculature. Preferably, the veins located in the lower extremities are treated via a small incision in a knee cavity.

It will be appreciated that an embodiment of a thrombectomy catheter comprising a capture cone is known from US 7, 220, 269. The known thrombectomy catheter comprises an occluder, i.e., an inflatable balloon arranged at the distal end of the catheter. Proximally to the occluder in a sub-distal region of the catheter the capture cone is positioned, which is arranged to stationary dwell at a selected position in a blood vessel. During use, the known catheter is positioned inside the blood vessel so that thrombus is positioned between the cone and the occluder. For purposes of removing thrombus, the occluder is displaced towards the capture cone thereby pushing thrombus into the capture cone for collecting thereof.

The thrombectomy catheter known from US 7, 220, 269 has the following disadvantages. First, low efficiency regarding removal of thrombus is obtainable, because the occluder is a smooth body which in fact may slip on the surface of thrombus by-passing it. Secondly, the known thrombectomy catheter may be not suited for treating elongated thrombi, because forces exerted by a substantially distantly located occluder may be not sufficient to cause thrombus to separate from inner periphery of the blood vessel and to penetrate the capture cone. Next, the capture cone is conceived to be manufactured separately and to be laminated to the distal end of the capture catheter which forms part of the known thrombectomy catheter. This may limit forces exertable on the capture cone to avoid delamination thereof on one hand, and leads to increase of manufacturing costs, on the other hand. Finally, the known thrombectomy catheter is not suitable for treating thrombi in lower extremities, because the occluder is to be displaced bi-directionally, namely in one direction during introduction thereof and in the opposite direction during pushing thrombus into the capture cone. Such bi-directional displacement may harm valves located in the veins of the lower extremities, leading to unacceptable medical complications.

In an embodiment of the thrombectomy catheter according to the invention the cross-section of the lumen in the expanded state is settable to a larger value than local cross-sectional dimensions of the blood vessel for stretching the blood vessel.

It is found that when the cross-section of the lumen of the thrombectomy catheter according to the invention is set to about 10% larger value than a local cross-section of the blood vessel a further increase of efficacy of removal of thrombus is obtained.

Preferably, the lumen is pre-tensioned for assisting transition between the collapsed state and the expanded state and/or for assisting is said stretching. This feature may be enabled by providing an expandable, substantially rigid body in the lumen. For example, a suitable wire or a wired structure may be used for this purpose. The wire or a wired structure may be integrated in a material of the lumen. Alternatively, the wire or the wired structure may be received by the lumen or may suitably be affixed to an inner surface of the lumen. Such wire or a wired structure may bias the lumen for exerting radial forces causing radial expansion of the lumen. In a collapsed state such forces may be not present, or may suitably be counteracted to prevent the lumen from expanding. Upon actuation causing a radial expansion of the lumen or upon removal of the counteracting forces the lumen undergoes transition to an expanded state.

In an embodiment of the thrombectomy catheter according to the invention the lumen comprises folded regions which are arranged to radially unfold for enabling a transition from the collapsed state to the expanded state.

This feature is based on the insight that it is preferable to provide a thrombectomy catheter having a substantially maximized cross-section of the distal end. It may be a case that the cross-section of an elastic lumen in it's expanded state may be limited by the elasticity of the material of the lumen, especially when the lumen forms an integral part of an overall material of the catheter. In order to enable still greater cross-sections of the lumen in comparison with those which may be obtainable by stretching, for example, a 2.33 mm (7F), 2.66 mm (8F), 3 mm (9F) or a 3.33 mm (10F) catheter, the lumen at the distal end may comprise a number of folded regions, which are conceived to be unfolded in the expanded state. For example, such regions may be arranged with an actuator, for example a shape memory alloy (SMA) wire, which may be conceived to be bent in use exerting radial forces on the folded regions and causing unfolding thereof. In this way, when the SMA wire is at rest the regions are at least partially folded and the distal end of the thrombectomy catheter is in its collapsed state. In use, when the wire is actuated, for example by means of application of an electric current, the wire undergoes a controllable bend causing the folded regions to radially unfold for enabling the expanded state with an increased cross-section. It will be appreciated that the lumen of the thrombectomy catheter comprising foldable regions may be manufacture from either stretchable or from non-stretchable material.

In an embodiment of the thrombectomy catheter according to the invention the lumen is arranged to cooperate with an actuatable unit for undergoing said alteration.

It is found to be preferable to alternate from the collapsed state to the expanded state of the lumen positioned at the distal end of the thrombectomy catheter using a suitable actuator. SMA materials may be used for this purpose, because they may be arranged to have two stable configurations, a first configuration corresponding to a collapsed state of the lumen and a second configuration corresponding to the expanded state of the lumen. Preferably, the lumen at least partially comprises the actuatable unit.

For example, the actuatable unit may relate to a cage or a wire-frame. Preferably, the actuatable unit comprises a cage or a wire-frame at least partially covered by a cover member, the cage or the wire-frame being collapsed when substantially covered by the cover member and being expanded when substantially exposed.

For example, the cover member may relate to a retractable catheter sheet. This embodiment has an advantage that no electric current is needed for actuating the cross-section of the lumen, eliminating electric hazard for a patient.

In an embodiment of the thrombectomy catheter according to the invention the wire-frame is partially received by the lumen, the wire-frame being cone-shaped at least in a region distally from the lumen.

It is found to be advantageous to provide the wire-frame distally to the lumen for assisting in protruding through thrombus. It will be appreciated that such wire frame does not hamper a substantially frontal penetration of the lumen by thrombus and, thus, does not decrease efficacy of the thrombectomy catheter. The extending cone of the wire-frame may be arranged with substantially sharpened wires for cutting through the thrombus, still further improving efficacy of the intervention.

In a still further embodiment of the thrombectomy catheter according to the invention at least an outer surface of the lumen conceived to contact the blood vessel comprises an anti-friction agent.

It is found to be advantageous to provide, preferably to coat, the outer surface of at least the distal end of the thrombectomy catheter with an anti-friction agent for further decreasing a mechanical resistance of the blood vessel to displacement of the catheter. This may lead to a decrease of damage risk for the inner periphery of the blood vessel, on one hand, and to increase of efficacy of the intervention, on the other hand. It will be appreciated that it lies within an ordinary skill of the artisan to select a suitable material for anti-friction agent.

In a still further embodiment of the thrombectomy catheter according to the invention the lumen further comprises a morcellator for at least partially destructing thrombus received by the lumen.

It is found to be advantageous to still further increase the efficacy of the thrombectomy intervention by providing the catheter with supplementary means for destroying thrombus. For example, the morcellator may comprise a suitable rotational body for mechanically cutting the tissue received by the lumen.

In a still further embodiment of the thrombectomy catheter according to the invention, the catheter is arranged with a drug deposition system conceived to act on thrombus.

For example, suitable chemical thrombolysis agents may be injected via a supplementary lumen of the catheter to at least partially dissolve the thrombotic tissue prior to or simultaneously with displacement of the lumen along the blood vessel.

A thrombectomy device according to the invention comprises a thrombectomy catheter as described with reference to the foregoing.

These and other aspects of the invention will be discussed in more detail with reference to the figures, wherein like reference signs correspond to like elements. It will be appreciated that figures are presented for illustrative purposes and may not be used for limiting the scope of the appended claims.

### BRIEF DESCRIPTION OF THE DRAWINGS

Figure 1 presents a schematic view of an embodiment of the thrombectomy catheter according to the invention.
Figure 2 presents a schematic view of a further embodiment of the thrombectomy catheter according to the invention.

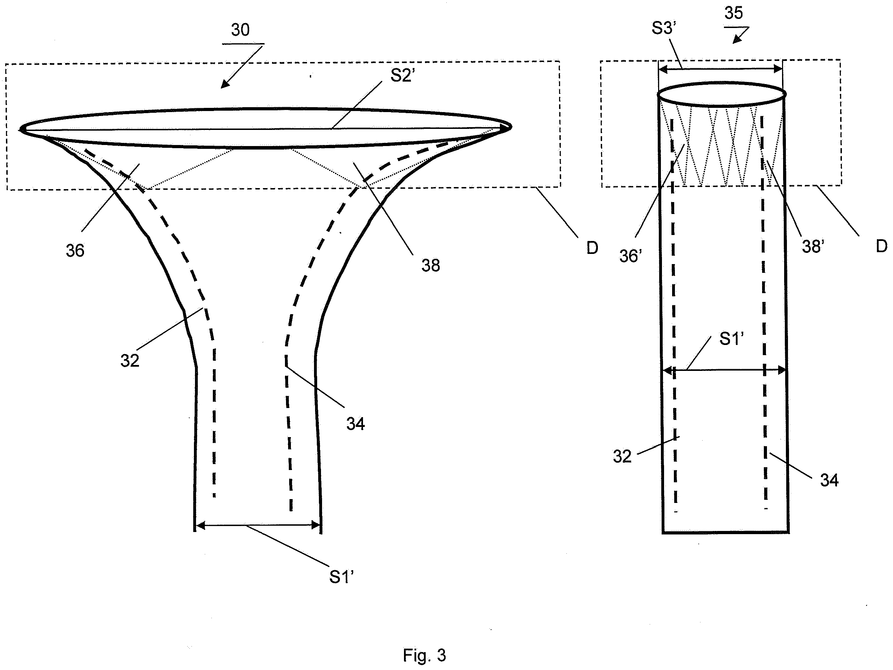

Figure 3 presents a schematic view of a still further embodiment of the thrombectomy catheter according to the invention.

### DETAILED DESCRIPTION OF THE DRAWINGS

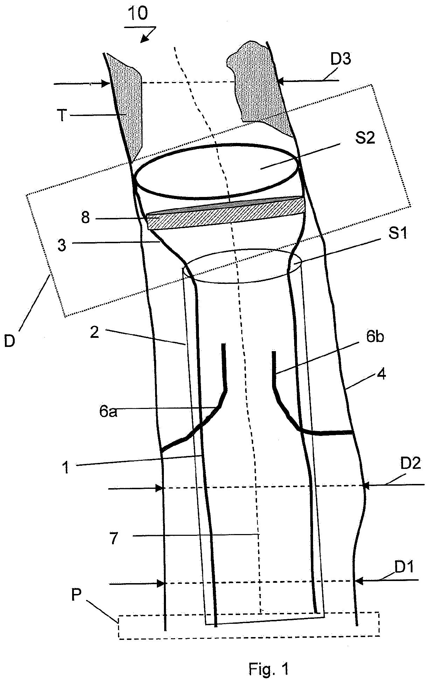

Figure 1 presents a schematic view of an embodiment of the thrombectomy catheter according to the invention. The thrombectomy catheter 10 may be used during minimally invasive thrombectomy, wherein the catheter is displaced along the inner periphery of a blood vessel 4, for example a lower extremity vein, to at least partially remove and collect thrombi T. The thrombectomy catheter 10 may be used for at least partially, preferably substantially, removing venous thrombus and/or deep venous thrombus.

The thrombectomy catheter 10 comprises a lumen 3 arranged at the distal end D of the catheter, wherein said lumen is alterable between a collapsed state (now shown) and an expanded state S2. For this purpose the lumen 3 may cooperate with an actuatable unit 8, which may be conceived to be collapsed at rest and to expand at use exerting radial forces to the lumen 3 causing radial expansion thereof. The actuatable unit 8 may relate to any suitable wire or a wire-frame configuration, comprising but not limited to a set of longitudinally arranged wire strips, a ring 8, a cage or any other suitable structure. Preferably, the actuatable unit is preserved at its collapsed condition at rest by the cover sheet 2 having a smaller cross-section S1. The actuactable unit 8 together with the lumen 3 may substantially be confined within the smaller cross-section S1. Preferably, the cross-section S1 corresponds to a standard intravascular cross-section corresponding to 2.33 - 3.33 mm (7F - 10F) catheters. It will be appreciated that invention may be practiced on catheters having different dimensions.

It will be appreciated that the thrombectomy catheter according to the invention having the increased cross-section S2 is conceived to be uni-directionally displaced along the blood vessel 4. In this case no damage is caused to the valves 6a, 6b present in the lower extremity veins. When the intervention is completed the cross-section of the lumen 3 is reduced to the original cross-section S1, for example by positioning the retractable cover sheet 2 over the distal end D of the catheter 10.

The thrombectomy catheter 10 comprises a tubular structure 1 running from a proximal end P to a distal end D and terminating at the distal end D with a lumen 3, which may be altered from a substantially collapsed cross-section S1 to a substantially extended cross-section S2. The lumen 3 forms preferably an integral part of the tubular structure 1.

In accordance with an aspect of the invention, the cross-section S2 of the lumen 3 at the distal portion D is conceived to be self-adjustable at least to substantially match cross-sectional dimensions D1, D2, D3, etc of the inner periphery of the blood vessel 4. It is found that next to a consistent difference in cross-sectional sizes between a proximal part of a vessel D1 and a distal part of the vessel D3, also local size differences D2 may occur. By arranging the distal portion 3 of the catheter with a lumen having an increased cross-sectional dimension S2, which is self-adjustable to a local cross-section of the blood vessel a substantially continuous contact between the lumen 3 and the inner periphery of the blood vessel 4 is maintained. In this way the lumen 3 may act as an efficient scoop for efficiently collecting thrombi T encountered by the lumen 3 along its displacement through the blood vessel 4. In order to facilitate said displacement a guide wire 7 may be provided.

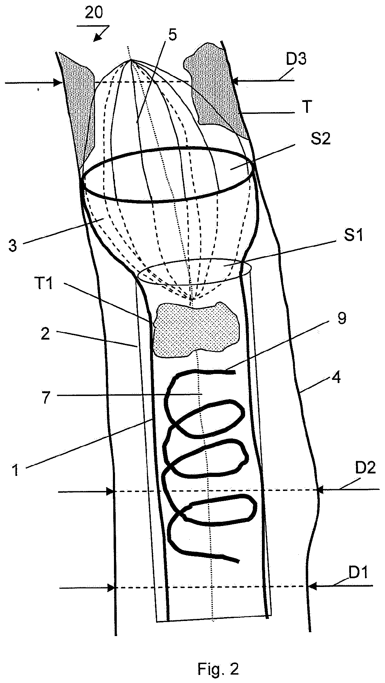

Figure 2 presents a schematic view of a further embodiment of the thrombectomy catheter according to the invention. In this particular embodiment the thrombectomy catheter, discussed with reference to Figure 1, the lumen 3 is provided with an actuator unit 5, which may be partially received by the lumen. The actuator 5 may relate to any suitable wire-frame comprising any shape memory alloy. In this case the actuator unit is arranged to assist the lumen 3 in maintaining the desired expanded cross-section S2, even when substantially obstructed blood vessels having substantially elongated thrombi T are to be treated.

Preferably, the actuator 5 has a cage form, allowing for a substantially frontal penetration the lumen 3 by thrombus. The cage 5 is collapsible when the cover sheet 2 is positioned substantially over the lumen 3. Due to the shape memory characteristic, the cage 5 will expand (as shown), when the cover sheet 2 is retracted exposing the cage 5. When thrombus T1 is received by the lumen 3 it may be destroyed by a morcellator 9 arranged proximally to the lumen 3. Any suitable morcellator known in the art may be suitable for this purpose.

## Claims

1. A thrombectomy catheter (10) comprising a distal end for at least partially removing thrombus from a blood vessel said distal end being conceived to undergo displacement along the blood vessel, wherein said distal end comprises: a lumen (3) arranged for collecting thrombus and having a cross-section which is alterable from a collapsed state to an expanded state, wherein the lumen is arranged to cooperate with an actuable unit (5, 8) for undergoing said alteration, and wherein
during said displacement the cross-section of the lumen in the expanded state is self-adjustable for substantially matching local cross-sectional dimensions of the blood vessel, **characterised in that** the lumen is pre-tensioned for assisting said expanding.

2. A catheter according to claim 1, wherein the catheter (10) comprises a proximal end and being formed from a tubular structure (1) propagating from the proximal end to the distal end, the lumen (3) forming integral part of said tubular structure.

3. A catheter according to claim 1 or 2, wherein said cross-section of the lumen (3) in the expanded state is settable to a larger value than said local cross-sectional dimensions for stretching the blood vessel.

4. A catheter according to claim 3, wherein the lumen (3) is pre-tensioned for assisting said stretching.

5. A catheter according to any one of the preceding claims, wherein the lumen (3) comprises folded regions which are arranged to radially unfold for enabling a transition from the collapsed state to the expanded state.

6. A catheter according to claim 1, wherein the lumen (3) at least partially comprises the actuatable unit.

7. A catheter according to claim 6, wherein the actuatable unit (5, 8) comprises a wire-frame at least partially covered by a cover member (2), the wire-frame being collapsed when substantially covered by the cover member and being expanded when substantially exposed.

8. A catheter according to claim 7, wherein the wire-frame comprises a shape memory alloy.

9. A catheter according to claim 7 or 8, wherein the wire -frame is partially received by the lumen (3), the wire-frame being cone-shaped at least in a region distally from the lumen.

10. A catheter according to any one of the preceding claims 7 - 9, wherein the cover member comprises a retractable cover sheet (2).

11. A catheter according to any one of the preceding claims, wherein at least an outer surface of the lumen (3) conceived to contact the blood vessel comprises an anti-friction agent.

12. A catheter according to any one of the preceding claims, wherein the lumen (3) further comprises a morcellator (9) for at least partially destructing thrombus received by the lumen.

13. A catheter according to any one of the preceding claims, further arranged with a drug deposition system conceived to act on thrombus.

14. A thrombectomy device comprising a thrombectomy catheter according to any one of the preceding claims.

## Patentansprüche

1. Thrombektomie-Katheter (10), umfassend ein distales Ende zum zumindest teilweisen Entfernen eines Thrombus aus einem Blutgefäß, wobei das distale Ende dazu ausgelegt ist, entlang des Blutgefäßes verschoben zu werden, wobei das distale Ende das Folgende umfasst: ein Lumen (3), das zum Auffangen des Thrombus angeordnet ist und einen Querschnitt aufweist, der von einem kollabierten Zustand in einen expandierten Zustand veränderbar ist, wobei das Lumen zum Zusammenwirken mit einer betätigbaren Einheit (5, 8) angeordnet ist, damit es diese Veränderung durchlaufen kann, wobei während der Verschiebung der Querschnitt des Lumens im expandierten Zustand selbstanpassbar ist, damit er an die örtlichen Querschnittsabmessungen des Blutgefäßes angepasst ist, **dadurch gekennzeichnet, dass** das Lumen zur Unterstützung der Expansion vorgespannt ist.

2. Katheter nach Anspruch 1, wobei der Katheter (10) ein proximales Ende umfasst und von einer röhrenförmigen Struktur (1) gebildet wird, die sich vom proximalen Ende zum distalen Ende fortsetzt, wobei das Lumen (3) einen einstückigen Bestandteil der röhrenförmigen Struktur bildet.

3. Katheter nach Anspruch 1 oder 2, wobei der Querschnitt des Lumens (3) im expandierten Zustand zum Dehnen des Blutgefäßes auf einen größeren Wert als die örtlichen Querschnittsabmessungen einstellbar ist.

4. Katheter nach Anspruch 3, wobei das Lumen (3) zur Unterstützung der Dehnung vorgespannt ist.

5. Katheter nach einem der vorhergehenden Ansprüche, wobei das Lumen (3) gefaltete Regionen umfasst, die dazu angeordnet sind, sich radial aufzufalten, um einen Übergang aus dem kollabierten Zustand in den expandierten Zustand zu ermöglichen.

6. Katheter nach Anspruch 1, wobei das Lumen (3) die betätigbare Einheit zumindest teilweise umfasst.

7. Katheter nach Anspruch 6, wobei die betätigbare Einheit (5, 8) einen Drahtrahmen umfasst, der zumindest teilweise von einem Abdeckelement (2) abgedeckt wird, wobei der Drahtrahmen kollabiert ist, wenn er von dem Abdeckelement im Wesentlichen abgedeckt wird, und expandiert ist, wenn er im Wesentlichen freiliegt.

8. Katheter nach Anspruch 7, wobei der Drahtrahmen eine Formgedächtnislegierung umfasst.

9. Katheter nach Anspruch 7 oder 8, wobei der Drahtrahmen teilweise vom Lumen (3) aufgenommen wird, wobei der Drahtrahmen zumindest in der Region distal vom Lumen kegelförmig ist.

10. Katheter nach einem der vorhergehenden Ansprüche 7-9, wobei das Abdeckelement eine zurückziehbare Abdeckplatte (2) umfasst.

11. Katheter nach einem der vorhergehenden Ansprüche, wobei zumindest eine äußere Oberfläche des Lumens (3), die für einen Kontakt mit dem Blutgefäß ausgelegt ist, ein reibungsminderndes Mittel umfasst.

12. Katheter nach einem der vorhergehenden Ansprüche, wobei das Lumen (3) ferner einen Morcellator (9) umfasst, um den vom Lumen aufgenommenen Thrombus zumindest teilweise zu zerstören.

13. Katheter nach einem der vorhergehenden Ansprüche, ferner angeordnet mit einem Arzneimitteldepositionssystem, das dazu ausgelegt ist, auf den Thrombus zu wirken.

14. Thrombektomie-Vorrichtung, umfassend einen Thrombektomie-Katheter nach einem der vorhergehenden Ansprüche.

## Revendications

1. Cathéter de thrombectomie (10) comprenant une extrémité distale pour retirer au moins partiellement un thrombus d'un vaisseau sanguin, ladite extrémité distale étant conçue pour subir un déplacement le long du vaisseau sanguin,
dans lequel ladite extrémité distale comprend une lumière (3) agencée pour ramasser le thrombus et présenter une section transversale qui est sujette à modification d'un état affaissé à un état dilaté,
dans lequel la lumière est agencée pour coopérer avec une unité (5, 8) pouvant être actionnée pour subir ladite modification, et
dans lequel pendant ledit déplacement la section transversale de la lumière dans l'état dilaté est autoréglable pour s'adapter sensiblement aux aires de section locales du vaisseau sanguin,
**caractérisé en ce que** la lumière est pré-tendue pour aider ladite expansion.

2. Cathéter selon la revendication 1, dans lequel le cathéter (10) comprend une partie proximale et est constitué à partir d'une structure tubulaire (1) se propageant de l'extrémité proximale à l'extrémité distale, la lumière (3) faisant partie intégrante de ladite structure tubulaire.

3. Cathéter selon la revendication 1 ou 2, dans lequel on peut régler la section transversale de la lumière (3) à une valeur supérieure aux dites aires de section locales pour élargir le vaisseau sanguin.

4. Cathéter selon la revendication 3, dans lequel la lumière (3) est pré-tendue pour aider ledit étirage.

5. Cathéter selon l'une quelconque des revendications précédentes, dans lequel la lumière (3) comprend des zones pliées qui sont agencées pour se déplier radialement afin de permettre une transition de l'état affaissé à l'état dilaté.

6. Cathéter selon la revendication 1, dans lequel la lumière (3) comprend au moins en partie l'unité pouvant être actionnée.

7. Cathéter selon la revendication 6, dans lequel l'unité (5, 8) pouvant être actionnée comprend une armature en fil couverte au moins partiellement par un élément de couverture (2), l'armature en fil étant affaissée quand elle est sensiblement couverte par l'élément de couverture et étant dilatée quand elle est sensiblement à découvert.

8. Cathéter selon la revendication 7, dans lequel l'armature en fil est composée d'un alliage à mémoire de forme.

9. Cathéter selon la revendication 7 ou 8, dans lequel l'armature en fil est partiellement reçue dans la lumière (3), l'armature en fil ayant une forme conique au moins dans une zone distale par rapport à la lumière.

10. Cathéter selon l'une quelconque des revendications 7 à 9, dans lequel l'élément de couverture comprend une feuille de couverture (2) escamotable.

11. Cathéter selon l'une quelconque des revendications précédentes, dans lequel au moins une surface extérieure de la lumière (3) conçue pour être en contact avec le vaisseau sanguin comprend un lubrifiant.

12. Cathéter selon l'une quelconque des revendications précédentes, dans lequel la lumière (3) comprend en outre un morcellateur (9) pour détruire au moins partiellement le thrombus recueilli dans la lumière.

13. Cathéter selon l'une quelconque des revendications précédentes, pourvu en outre d'un système de dépôt de médicament conçus pour agir sur le thrombus.

14. Dispositif de thrombectomie comprenant un cathéter de thrombectomie selon l'une quelconque des revendications précédentes.
